# EUROPEAN PATENT APPLICATION

(11) **EP 2 018 866 A1**
(43) Date of publication of application: **28.01.2009**
(21) Application number: 07113349.0
(22) Date of filing: 27.07.2007
(51) Int. Cl.: A61K 31/506, A61K 47/48, A61K 9/08, A61P 31/10

(54) **Pharmaceutical compositions containing voriconazole**

(71) Applicant: SANDOZ AG, 4056 Basel (CH)
(72) Inventor: Welz, Christian, 6230 Brixlegg (AT)
(74) Representative: Kröger, Bernd

(57) **Abstract**

The present invention provides a process for improving the solubility of voriconazole in aqueous solutions. The present invention also provides a composition comprising voriconazole and a beta-cyclodextrin obtainable by this process, a method of treating a fungal infection, a pharmaceutical composition comprising the composition comprising voriconazole and a beta-cyclodextrin and the use of a therapeutically effective amount of the composition comprising voriconazole and a beta-cyclodextrin and at least one pharmaceutically acceptable excipient for the preparation of a medicament for treating a fungal infection.

## Description

### Field of the Invention

The present invention relates to a process for improving the solubility of voriconazole in aqueous solutions, and compositions comprising voriconazole and a beta-cyclodextrin, which may be used in pharmaceutical compositions.

### Background of the Invention

(2R,3S)-2-(2,4-Difluorophenyl)-3-(5-fluoro-4-pyrimidinyl)-1-(1H-1,2,4-triazol-1-yl)-butan-2-ol, also known as voriconazole, is disclosed in EP-A 440 372. Voriconazole is useful in the treatment of fungal infections. Voriconazole has a low aqueous solubility (0.61 mg/ml at a pH 7; 0.2 mg/ml at a pH 3), and is not stable in water. Thus, development of an aqueous intravenous formulation with a sufficient shelf life is difficult. These problems are magnified by the semi-polar nature of the compound (log D=1.8) which means that it is not generally solubilised by conventional means such as oils, surfactants or water miscible co-solvents.

For solid drugs it is also important to render the sparingly water-soluble drug water-soluble since a good solubility increases the bioavailability of the drug. It has been described that inclusion compounds, e.g. with urea or complexes of polyvinyl pyrrolidone may improve the solubility of a compound but in aqueous solution they are not stable. Such inclusion compounds are therefore at best suitable for solid application forms of drugs.

WO 98/58677 discloses that the solubility of voriconazole in water can be increased by molecular encapsulation with sulphoalkylether cyclodextrin derivatives of the type disclosed in WO 91/11172, particularly beta-cyclodextrin derivatives wherein the cyclodextrin ring is substituted by sulphobutyl groups. There are complex manufacturing issues associated with cyclodextrin formulations that significantly increase cost of goods.

WO 97/28169 discloses a phosphate pro-drug of voriconazole, which exhibits increased solubility and aqueous stability. However, the pro-drug does not exhibit 100% bioequivalence to voriconazole, such that cost of goods is again significantly increased.

US 2005/112204 relates to a new pharmaceutical formulation of voriconazole, in particular a formulation comprising one or more poloxamers.

US 6,632,803 provides pharmaceutical formulation comprising voriconazole, or a pharmaceutically acceptable derivative thereof, and a sulfobutylether beta-cyclodextrin.

WO 2004/032902 relates to compositions of an aqueous suspension of submicron- to micron-size particles of an antifungal agent coated with one or more surfactants. The particles of the antifungal agent should have a volume-weighted mean particle size of less than about 50 µm in diameter. In one embodiment, the antifungal agent is a triazole antifungal agent such as itraconazole, ketoconazole, miconazole, fluconazole, ravuconazole, voriconazole, saperconazole, eberconazole, genaconazole, and posaconazole.

US 5,773,443 relates to triazole derivatives which have antifungal activity, more particularly to 2-aryl-3-(3-halopyridin-4-yl or 5-halopyrimidin-4-yl)-1-(1H-1,2,4-triazol-1-yl)alkan-2-ol derivatives which are useful in the treatment of fungal infections in animals, including human beings.

US 5,116,844 relates to novel triazole derivatives which have antifungal activity and are useful in the treatment of fungal infections in animals, including humans.

EP 0 149 197 discloses pharmaceutical compositions which comprise inclusion compounds of drugs, which are instable or only sparingly soluble in water, with partially etherified beta-cyclodextrin derivatives having hydroxyalkyl and optionally additional alkyl groups.

Accordingly there is a need for formulations of voriconazole that increase its solubility and aqueous stability.

### Summary of the Invention

This invention in general relates to pharmaceutical compositions comprising voriconazole, as well as processes for the preparation of such compositions.

The present invention provides a process for improving the solubility of voriconazole in aqueous solutions comprising
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

Preferably the temperature in step (ii) is in the range of from 40 °C to 60 °C.

According to a further embodiment, the present invention also provides a process for improving the solubility of voriconazole in aqueous solutions as described above, wherein in step (ii) the solution comprising voriconazole and a beta-cyclodextrin is kept at a temperature of at least 40 °C for a time of from 1 minute to 24 hours.

According to a further embodiment, the present invention also provides a process for improving the solubility of voriconazole in aqueous solutions as described above, wherein the process comprises an additional step (iii):
(iii) lyophilisation of the composition obtained in step (ii).

In particular, the molar ratio of voriconazole and a beta-cyclodextrin in the composition is in the range of from 2.5 to 3.75.

According to the present invention the beta-cyclodextrin is preferably selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins, more preferably, the beta-cyclodextrin is a hydroxyalkylated beta-cyclodextrin such as a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

According to another aspect, the present invention also provides a process for preparing a composition comprising voriconazole and a beta-cyclodextrin, said process comprising the steps of
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

According to another aspect, the present invention also provides a composition comprising voriconazole and a beta-cyclodextrin obtainable by a process as described above.

Preferably, the molar ratio of voriconazole and a beta-cyclodextrin in the composition according to the present invention is in the range of from 2.5 to 3.75.

Preferably, the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins, more particular wherein the beta-cyclodextrin is a hydroxyalkylated beta-cyclodextrin, more preferably, the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

The present invention also provides a method of treating a fungal infection comprising administering to a patient in need of such treating a therapeutically effective amount of the composition comprising voriconazole and a beta-cyclodextrin according to the present invention or a composition obtainable according to a process according to the present invention and at least one pharmaceutically acceptable excipient.

According to a further aspect, the present invention also provides a pharmaceutical composition comprising the composition comprising voriconazole and a beta-cyclodextrin according to the present invention or a composition obtainable according to a process according to the present invention and at least one pharmaceutically acceptable excipient.

Preferably, the pharmaceutical composition is a pharmaceutical composition for treating a fungal infection in a mammal, in particular in man.

According to another aspect, the present invention also provides the use of a therapeutically effective amount of the composition comprising voriconazole and a beta-cyclodextrin according to the present invention or a composition obtainable according to a process according to the present invention and at least one pharmaceutically acceptable excipient for the preparation of a medicament for treating a fungal infection, preferably wherein said medicament is to be administered to a patient in need thereof.

### Description of the Invention

This invention in general relates to pharmaceutical compositions comprising voriconazole, as well as processes for the preparation of such compositions.

The present invention provides a process for improving the solubility of voriconazole in aqueous solutions comprising
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

It has been found that the solubility of voriconazole in an aqueous medium may be improved by complexation with a beta-cyclodextrin, in particular a hydroxyalkyl derivative of a beta-cyclodextrin, in the preparation of an appropriate pharmaceutical composition. The complexation of voriconazole with a beta-cyclodextrin can be improved by keeping the solution comprising voriconazole and a beta-cyclodextrin at a temperature of at least 40 °C.

According to the present invention, a composition comprising voriconazole and a beta-cyclodextrin is prepared by a process comprising the preparation of an aqueous solution of voriconazole and a beta-cyclodextrin and then keeping the solution obtained in step (i) comprising voriconazole and a beta-cyclodextrin at a temperature of at least 40 °C to obtain a composition comprising voriconazole and a beta-cyclodextrin. According to this process, the formation of a complex between voriconazole and the beta-cyclodextrin is improved and the amount of voriconazole which can be dissolved is increased. The complexation is improved by step (ii) of the process of the present invention in comparison to a complexation which is carried out at room temperature.

It has been found that keeping the solution obtained in step (i) comprising voriconazole and a beta-cyclodextrin at a temperature of at least 40 °C improves the complexation and that the complex of voriconazole and the beta-cyclodextrin which is at least partially formed in step (ii) is also stable when the composition is cooled to room temperature after step (ii).

In the context of the present invention, an aqueous solution is a solution which comprises water but can also comprise other solvents, in particular solvents which are miscible with water.

In the context of the present invention, the term beta-cyclodextrin is used for beta-cyclodextrin and derivatives thereof.

According to the present invention, in a first step (i) an aqueous solution of voriconazole and a beta-cyclodextrin is prepared. According to the present invention, the preparation can be carried out in any fashion known to the person skilled in the art. It is possible to dissolve voriconazole first in an aqueous solution and then add the beta-cyclodextrin. It is also possible to first dissolve the beta-cyclodextrin in an aqueous solution and then add voriconazole.

According to the present invention, step (i) can be carried out at any suitable temperature, for example at room temperature or at elevated temperature.

According to step (ii) of the process according to the present invention, the solution obtained in step (i) comprising voriconazole and a beta-cyclodextrin is kept at a temperature of at least 40°C to obtain a composition comprising voriconazole and a beta-cyclodextrin. During step (ii) at least partially a complex of voriconazole and the beta-cyclodextrin is formed.

According to the present invention, the temperature in step (ii) is preferably at least 45°C, more preferably at least 50 °C, in particular at least 55 °C. The step (ii) of the present invention can be for example carried out at a temperature in the range of from 40 °C to 60 °C, in particular from 45 °C to 55 °C.

Therefore, according to a further embodiment, the present invention also provides a process for improving the solubility of voriconazole in aqueous solutions as described above, wherein the temperature in step (ii) is in the range of from 40 °C to 60 °C.

According to the present invention, step (ii) of the process according to the present invention is carried out for a time sufficient to form at least partially a complex between voriconazole and the beta-cyclodextrin. For example, the solution comprising voriconazole and a beta-cyclodextrin is kept at a temperature of at least 40 °C for a time of from 1 minute to 24 hours according to step (ii), preferably for a time of from 5 minutes to 10 hours, in particular for a time of from 10 minutes to 5 hours, more preferably of from 15 minutes to 60 minutes, for example of from 30 minutes to 45 minutes.

According to a further embodiment, the present invention therefore also provides a process for improving the solubility of voriconazole in aqueous solutions as described above, wherein in step (ii) the solution comprising voriconazole and a beta-cyclodextrin is kept at a temperature of at least 40 °C for a time of from 1 minute to 24 hours.

According to the present invention, step (ii) can be carried out directly after step (i). It is also possible that between steps (i) and (ii) further steps are carried out in the context of the present invention.

The process of the present invention can also comprise further steps such as heating or cooling steps, in particular a step of heating the solution obtained in step (i) to the temperature of step (ii) or a step of cooling the composition obtained in step (ii) to room temperature.

The process of the present invention can also comprise other steps of treating the composition obtained in step (ii). It is for example possible to add further compounds to the composition obtained in step (ii). It is also possible, that for example a lyophilisation step is carried out after step (ii).

According to a further embodiment, the present invention also provides a process for improving the solubility of voriconazole in aqueous solutions as described above, wherein the process comprises an additional step (iii):
(iii) lyophilisation of the composition obtained in step (ii).

Step (iii) can be carried out directly after step (ii). It is also possible, that additional steps are carried out between step (ii) and step (iii).

It has been found that the stability of the voriconazole beta-cyclodextrin derivative complex is further enhanced by lyophilisation (freeze-drying).

The cyclodextrin derivatives used in compositions according to the invention enable the finished lyophilised product to accommodate high levels of moisture without a detrimental effect on stability.

According to the present invention, voriconazole can be used as such or in form of pharmaceutically acceptable salts, solvates, or derivatives thereof. According to the present invention derivatives include complexes, prodrugs and isotopically-labelled compounds, as well as salts and solvates thereof.

Pharmaceutically acceptable salts of voriconazole include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of voriconazole may be readily prepared by mixing together solutions of voriconazole and the desired acid or base, as appropriate. The salt may precipitate from solution and be collected by filtration or may be recovered by evaporation of the solvent. The degree of ionisation in the salt may vary from completely ionised to almost non-ionised.

Voriconazole or a pharmaceutically acceptable salt or derivative thereof may exist in both unsolvated and solvated forms. The term 'solvate' is used herein to describe a molecular complex comprising voriconazole, or a pharmaceutically acceptable salt or derivative thereof, and one or more pharmaceutically acceptable solvent molecules, for example, ethanol. The term 'hydrate' is employed when said solvent is water.

All references herein to voriconazole include references to salts thereof, and to solvates of voriconazole or salts thereof.

The compositions of the invention include voriconazole as hereinbefore defined, polymorphs and prodrugs thereof and isotopically-labelled voriconazole or a pharmaceutically acceptable salt or solvate thereof.

As stated, the invention includes all polymorphs and so-called 'prodrugs' of voriconazole. In respect of the latter, certain derivatives of voriconazole which may have little or no pharmacological activity themselves can, when administered into or onto the body, be converted into voriconazole, for example, by hydrolytic cleavage. Such derivatives are referred to as 'prodrugs'.

Prodrugs in accordance with the invention can, for example, be produced by replacing appropriate functionalities present in voriconazole with certain moieties known to those skilled in the art as 'pro-moieties'.

Voriconazole contains an alcohol functionality (-OH), therefore some examples of prodrugs in accordance with the invention may include an ester or ether thereof, for example, by replacement of the hydrogen by phosphorylation to provide (2R,3S)-2-(2,4-Difluorophenyl)-3-(5-fluoro-4-pyrimidinyl)-1-(1H-1,2,4-triazol-1-yl)-2-butyl dihydrogen phosphate as described in WO 97/28169. Further examples of replacement groups in accordance with the foregoing examples and examples of other prodrug types in accordance with the invention may be found in the aforementioned references.

The present invention also includes isotopically-labelled voriconazole or a pharmaceutically acceptable salt, solvate or derivative thereof, wherein one or more atoms are replaced by atoms having the same atomic number, but an atomic mass or mass number different from the atomic mass or mass number usually found in nature.

According to the present invention, the solubility of voriconazole in aqueous solution is increased, in particular the amount of voriconazole dissolved relative to the amount of the beta-cyclodextrin used. According to the present invention, the molar ratio of voriconazole and a beta-cyclodextrin is preferably in the range of from 2.5 to 3.75, more preferably in the range of from 2.75 to 3.5, in particular in the range of from 3.0 to 3.25.

Therefore, according to a further embodiment, the present invention also provides a process for improving the solubility of voriconazole in aqueous solutions as described above, wherein the molar ratio of voriconazole and a beta-cyclodextrin in the composition is in the range of from 2.5 to 3.75.

According to the present invention, it is possible to obtain compositions comprising voriconazole and a beta-cyclodextrin, which contain for example from about 40 mg/ml voriconazole to about 80 mg/ml voriconazole, preferably from about 45 mg/ml voriconazole to about 75 mg/ml voriconazole, in particular from about 50 mg/ml voriconazole to about 70 mg/ml voriconazole.

According to the present invention, voriconazole is mixed with a beta-cyclodextrin. Any suitable beta-cyclodextrin derivative can be used in the process of the present invention, Preferably, the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins, more preferably the beta-cyclodextrin is a hydroxyalkylated beta-cyclodextrin.

According to a preferred embodiment, the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

Therefore, according to a further embodiment, the present invention also provides a process for improving the solubility of voriconazole in aqueous solutions as described above, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins.

According to a further embodiment, the present invention also provides a process for improving the solubility of voriconazole in aqueous solutions as described above, wherein the beta-cyclodextrin is a hydroxyalkylated beta-cyclodextrin.

According to a further embodiment, the present invention also provides a process for improving the solubility of voriconazole in aqueous solutions as described above, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

According to another aspect, the present invention also provides a process for preparing a composition comprising voriconazole and a beta-cyclodextrin, said process comprising the steps of
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

With regard to the steps of the process and additional steps as well as the process conditions and the compounds used in this process, reference is made to the above description of the process for improving the solubility of voriconazole in aqueous solutions.

Therefore, according to another embodiment, the present invention also provides a process for preparing a composition comprising voriconazole and a beta-cyclodextrin as described above, wherein the temperature in step (ii) is in the range of from 40 °C to 60 °C.

According to another embodiment, the present invention also provides a process for preparing a composition comprising voriconazole and a beta-cyclodextrin as described above, wherein the molar ratio of voriconazole and a beta-cyclodextrin in the composition is in the range of from 2.5 to 3.75.

According to another embodiment, the present invention also provides a process for preparing a composition comprising voriconazole and a beta-cyclodextrin as described above, wherein in step (ii) the solution comprising voriconazole and a beta-cyclodextrin is kept at a temperature of at least 40 °C for a time of from 1 minute to 24 hours.

According to another embodiment, the present invention also provides a process for preparing a composition comprising voriconazole and a beta-cyclodextrin as described above, wherein the process comprises an additional step (iii):
(iii) lyophilisation of the composition obtained in step (ii).

According to another embodiment, the present invention also provides a process for preparing a composition comprising voriconazole and a beta-cyclodextrin as described above, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins.

According to another embodiment, the present invention also provides a process for preparing a composition comprising voriconazole and a beta-cyclodextrin as described above, wherein the beta-cyclodextrin is a hydroxyalkylated beta-cyclodextrin.

According to another embodiment, the present invention also provides a process for preparing a composition comprising voriconazole and a beta-cyclodextrin as described above, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

The present invention also relates to the compositions which can be obtained by any process as described above. Therefore, according to another aspect, the present invention also provides a composition comprising voriconazole and a beta-cyclodextrin obtainable by a process as described above.

Again, reference is made to preferred embodiments described in the context of the process of the present invention. In particular, reference is made to the explanation regarding voriconazole and the beta-cyclodextrin.

Therefore, according to a further embodiment, the present invention also provides a composition comprising voriconazole and a beta-cyclodextrin obtainable by a process as described above, wherein the molar ratio of voriconazole and a beta-cyclodextrin in the composition is in the range of from 2.5 to 3.75.

Therefore, according to a further embodiment, the present invention also provides a composition comprising voriconazole and a beta-cyclodextrin as described above, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins, more particular wherein the beta-cyclodextrin is a hydroxyalkylated beta-cyclodextrin.

According to a further embodiment, the present invention also provides a composition comprising voriconazole and a beta-cyclodextrin as described above, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

The compositions obtained in a process according to the present invention or the compositions of the present invention can be used as antifungal agents. The compositions according to the present invention are suitable for the use in pharmaceutical compositions.

In a further aspect the invention therefore provides a method of treating a fungal infection comprising administering to a patient in need of such treating a therapeutically effective amount of the composition comprising voriconazole and a beta-cyclodextrin according to the present invention or a composition obtainable according to a process according to the present invention and at least one pharmaceutically acceptable excipient.

In a further aspect the present invention also provides the use of a therapeutically effective amount of the composition comprising voriconazole and a beta-cyclodextrin according to the present invention or a composition obtainable according to a process according to the present invention and at least one pharmaceutically acceptable excipient for the preparation of a medicament for treating a fungal infection, preferably wherein said medicament is to be administered to a patient in need thereof.

Fungal infections which may be treated by voriconazole have been extensively described in the literature, including EP-A 440 372. For example, they are useful in treating topical fungal infections in man caused by, among other organisms, species of Candida, Trichophyton, Microsporum or Epidermophyton, or in mucosal infections caused by Candida albicans (e.g. thrush and vaginal cadidiasis). They can also be used in the treatment of systemic fungal infections caused by, for example, species of Candida (e.g. Candida albicans), Cryptococcus neoformans, Aspergillus flavus, Aspergillus fumigatus, Coccidioides, Paracoccidioides, Histoplasma or Blastomyces. It will be appreciated that reference to treatment is intended to include prophylaxis as well as the alleviation of established symptoms.

Likewise, EP-A 440 372 also describes a suitable dose for the parenteral administration of voriconazole. A typical dose is between 0.1 to 25 mg/kg, such as 0.5 to 20 mg/kg, for example 1 to 10 mg/kg, administered twice a day. It will be appreciated that the precise therapeutic dose of voriconazole will depend on the age and condition of the patient and the nature of the condition to be treated and will be at the ultimate discretion of the attendant physician.

According to a further aspect, the present invention also provides a pharmaceutical composition comprising the composition comprising voriconazole and a beta-cyclodextrin according to the present invention or a composition obtainable according to a process according to the present invention and at least one pharmaceutically acceptable excipient.

Preferably, the pharmaceutical composition is a pharmaceutical composition for treating a fungal infection in a mammal, in particular in man.

Compositions of the present invention suitable for parenteral administration need very few constituents, but it is generally preferred that an injectable solution is made up with saline to provide a solution which is iso-osmotic with blood. These compositions can be sterilised after preparation. They can be provided in any suitable form and in any suitable containers appropriate to maintaining sterility.

The present invention is suitable for pharmaceutical use. The compositions can be administered by various routes. Preferred routes of administration are parenteral and oral.

Modes of parenteral administration include intravenous, intra-arterial, intrathecal, intraperitoneal, intraocular, intra-articular, intramuscular, subcutaneous injection, and the like. The present invention may also be administered via other routes that include oral, buccal, periodontal, rectal, nasal, pulmonary, transdermal, or topical. In an embodiment of the present invention, the aqueous medium of the composition is removed to form dry particles. The method to remove the aqueous medium can be any method known in the art. One example is evaporation. Another example is freeze drying or lyophilization. The dry particles may then be formulated into any acceptable physical form including, but is not limited to, solutions, tablets, capsules, suspensions, creams, lotions, emulsions, aerosols, powders, incorporation into reservoir or matrix devices for sustained release (such as implants or transdermal patches), and the like. Administration routes of these pharmaceutical forms include, but are not limited to parenteral, oral, buccal, periodontal, rectal, nasal, pulmonary, transdermal and topical. Furthermore, the active pharmaceutical agent may be delivered using controlled or sustained release formulations, incorporation into delivery.

Preferably, the composition is for parenteral administration, for example, i.v. administration.

Generally, in aqueous intravenous and intramuscular formulations according to the invention, the voriconazole will be present at a concentration of from 5 mg/ml to 50 mg/ml, for example 10 mg/ml to 30 mg/ml. The beta-cyclodextrin derivative will be present in a molar ratio of voriconazole:cyclodextrin derivative of from 1:1 to 1:10, for example 1:2 to 1:7, in particular 1:2 to 1:3. The formulations may be lyophilised (freeze dried) for storage prior to use, and made up with water when required.

For human use, voriconazole and its salts can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs, solutions or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of a formulation comprising voriconazole and its salts will be from 0.01 to 20 mg/kg (in single or divided doses) when administered by either the oral or parenteral route. Thus tablets or capsules of the compounds will contain from 5 mg to 0.5 g of active compound for administration singly or two or more at a time, as appropriate. The above dosages are exemplary of the average case; there can, of course, be individual instances where higher or lower dosage ranges are merited, and such are within the scope of this invention.

Alternatively, the voriconazole can be administered in the form of a suppository or pessary, or they may be applied topically in the form of a lotion, solution, cream, ointment or dusting powder. For example, it can be incorporated into a cream consisting of an aqueous emulsion of polyethylene glycols or liquid paraffin; or it can be incorporated, at a concentration between 1 and 10%, into an ointment consisting of a white wax or white soft paraffin base together with such stabilizers and preservatives as may be required.

It is generally preferred that compositions of the present invention are provided in a form suitable for direct injection. It will be readily appreciated by those skilled in the art how to administer compositions of the present invention to a human or animal.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

Formulations for parenteral administration may be formulated to be immediate and/or modified release. Modified release formulations include delayed-, sustained-, pulsed-, controlled-, targeted and programmed release. Thus compositions of the invention may be formulated as a solid, semi-solid, or thixotropic liquid for administration as an implanted depot providing modified release of the active compound. Examples of such formulations include drug-coated stents and PGLA microspheres.

The compositions of the invention may also be administered topically to the skin or mucosa, that is, dermally or transdermally. Typical formulations for this purpose include gels, hydrogels, lotions, solutions, creams, ointments, dressings, foams, films, skin patches, wafers, implants, sponges, fibres, bandages and microemulsions. Liposomes may also be used. Typical carriers include alcohol, water, mineral oil, liquid petrolatum, white petrolatum, glycerine, polyethylene glycol and propylene glycol. Penetration enhancers may be incorporated.

Other means of topical administration include delivery by electroporation, iontophoresis, phonophoresis, sonophoresis and microneedle or needle-free (e.g. Powderject(TM), Bioject(TM), etc.) injection.

Formulations for topical administration may be formulated to be immediate and/or modified release. Modified release formulations include delayed-, sustained-, pulsed-, controlled-, targeted and programmed release.

The compositions of the invention can also be administered intranasally or by inhalation, typically as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane.

The pressurised container, pump, spray, atomizer, or nebuliser contains a solution of the compositions of the invention comprising, for example, ethanol (optionally, aqueous ethanol) or a suitable alternative agent for dispersing, solubilising, or extending release of the active, the propellant(s) as solvent and an optional surfactant, such as sorbitan trioleate, oleic acid, or an oligolactic acid.

A suitable solution formulation for use in an atomiser using electrohydrodynamics to produce a fine mist may contain from 1 µg to 20 mg of voriconazole or pharmaceutically acceptable salts, solvates, or derivatives thereof per actuation and the actuation volume may vary from 1 µl to 100 µl. A typical formulation may comprise a formulation of the invention, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly-DL-lactic-coglycolic acid (PGLA). Modified release formulations include delayed-, sustained-, pulsed-, controlled-, targeted and programmed release.

In the case of aerosols, the dosage unit is determined by means of a valve which delivers a metered amount. Units in accordance with the invention are typically arranged to administer a metered dose or "puff" containing from 1 µg to 10 mg of voriconazole or pharmaceutically acceptable salts, solvates, or derivatives thereof. The overall daily dose will typically be in the range 1 µg to 200 mg which may be administered in a single dose or, more usually, as divided doses throughout the day.

The formulations of the invention may also be administered directly to the eye or ear, typically in the form of drops of solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The following examples illustrate the process of the present invention and are not intended to limit the scope of the invention set forth in the claims appended thereto.

### Examples

The following substances were used:
Voriconazole, Matrix Laboratories Limited, Secunderabad, India, batch-number VRZ 0091004
Cavasol^{®} W7 HP Pharma, Wacker-Chemie GmbH, Burghausen, Germany, batch-number 73B011 (HPβCD)
Ultrapure water, Millipore Gradient A10 with UV-lamp and ultrafilter, Millipore, Molsheim, France (Corresponding to Wfl Ph. Eur. & USP)

### Example 1: Preparation of Hydroxypropyl-beta-cyclodextrin (HPβCD)-solutions

75.95 g HPβCD (water content: 1.2 %) were weighed into a beaker glass. 74.12 g of ultrapure water were added and HPβCD was dissolved using a magnetic stirrer to give a solution 1 with a HPβCD-concentration of 50 g/100 g.

20 g of solution 1 were mixed with 5 g ultrapure water to give a solution 2 with a HPβCD-concentration of 40 g /100 g.

81 g of solution 1 were mixed with 54 g ultrapure water to give a solution 3 with a HPβCD-concentration of 30 g /100 g.

80 g of solution 3 were mixed with 40 g ultrapure water to give a solution 4 with a HPβCD-concentration of 20 g /100 g.

10 g of solution 3 were mixed with 20 g ultrapure water to give a solution 5 with a HPβCD-concentration of 10 g / 100 g.

5 g of solution 3 were mixed with 20 g ultrapure water to give a solution 6 with a HPβCD-concentration of 5 g / 100 g.

80 - 100 mg Voriconazole were weighed into individual 2 mL Eppendorf-Caps.

1 mL of solution 1 to solution 6 were added to individual Eppendorf-Caps with Voriconazole. For comparison, as solution 7, ultrapure water was added to the Eppendorf-Cap with Voriconazole. The solutions were incubated in Eppendorf-shaker at defined temperature for approximately 20 hours.

### Example 1.1:

For example 1.1, the solutions were incubated in Eppendorf-shaker at 5°C for approximately 20 hours to give solutions 1 a to 7a.

### Example 1.2:

For example 1.2, the solutions were incubated in Eppendorf-shaker at 20 to 25 °C for approximately 20 hours to give solutions 1 b to 7b.

### Example 1.3:

For example 1.3, the solutions were incubated in Eppendorf-shaker at 40 °C for approximately 20 hours to give solutions 1 c to 7c.

### Example 1.4:

For example 1.4, the solutions were incubated in Eppendorf-shaker at 60 °C for approximately 20 hours to give solutions 1 d to 7d.

After incubation, solutions were filtered using a 0.45 µm syringe filter. No formation of precipitates was observed due to temperature change during filtration.

### Example 2: Determination of Assay of Voriconazole of filtered solutions

The solutions obtained according to example 1 were further diluted after 0.45 µm filtration to obtain an absorbance which is within linear range of calibration curve for UV-determination of Voriconazole assay. Determinations were performed twofold, generally.

Assay of Voriconazole was determined by UV-spectroscopy. Briefly, absorbance was measured at 256 nm using a 0.1 cm cuvette. A calibration curve using Voriconazole batch-number VRZ 0091004 was recorded by determination of absorbance of 6 different Voriconazole solutions. Absorbance was in a range of 0.22 to 1.35.

### Example 2.1:

For a temperature of 5 °C, the following results according to table 1 were obtained:

**Table 1**

| Solution | Concentration HPβCD, | Concentration Voriconazole, | absorption |
|---|---|---|---|
| | [g/g] | [mg/ml] | |
| 1a | 50/100 | 6.80 | 0.14969 |
| 2a | 40/100 | 34.75 | 0.76455 |
| 3a | 30/100 | 27.56 | 0.96246 |
| 4a | 20/100 | 18.97 | 1.0434 |
| 5a | 10/100 | 10.01 | 0.9177 |
| 6a | 5/100 | 5.55 | 1.0168 |
| 7a | 0/100 | 0.66 | 0.72324 |

### Example 2.2:

For a temperature of 20 °C, the following results according to table 2 were obtained:

**Table 2**

| Solution | Concentration HPβCD, | Concentration Voriconazole, | absorption |
|---|---|---|---|
| | [g/g] | [mg/ml] | |
| 1b | 50/100 | 16.90 | 0.09243 |
| 2b | 40/100 | 38.54 | 0.84309 |
| 3b | 30/100 | 30.86 | 1.0715 |
| 4b | 20/100 | 20.40 | 1.1159 |
| 5b | 10/100 | 11.05 | 1.0075 |
| 6b | 5/100 | 5.89 | 1.0743 |
| 7b | 0/100 | 0.71 | 1.5568 |

### Example 2.3:

For a temperature of 40 °C, the following results according to table 3 were obtained:

**Table 3**

| Solution | Concentration HPβCD, | Concentration Voriconazole, | absorption |
|---|---|---|---|
| | [g/g] | [mg/ml] | |
| 1c | 50/100 | 57.74 | 1.2701 |
| 2c | 40/100 | 47.69 | 1.049 |
| 3c | 30/100 | 35.64 | 1.2446 |
| 4c | 20/100 | 23.64 | 1.3001 |
| 5c | 10/100 | 12.17 | 1.1153 |
| 6c | 5/100 | 6.53 | 1.1649 |
| 7c | 0/100 | 0.86 | 0.94327 |

### Example 2.4:

For a temperature of 60 °C, the following results according to table 4 were obtained:

**Table 4**

| Solution | Concentration HPβCD, | Concentration Voriconazole, | absorption |
|---|---|---|---|
| | [g/g] | [mg/ml] | |
| 1d | 50/100 | 71.77 | 0.79349 |
| 2d | 40/100 | 56.48 | 0.62127 |
| 3d | 30/100 | 41.32 | 1.0821 |
| 4d | 20/100 | 27.51 | 1.0257 |
| 5d | 10/100 | 14.21 | 0.97721 |
| 6d | 5/100 | 7.72 | 0.70722 |
| 7d | 0/100 | 1.50 | 1.6475 |

The data show a linear relation for all temperatures up to 40 % HPβCD-concentration. For 5 °C and 20 °C incubation temperature, Voriconazole cannot be incorporated into cyclodextrin and therefore concentration of Voriconazole which is incorporated into HPβCD by inclusion complex formation is reduced.

The amount of Voriconazole incorporated into HPβCD is strongly dependent on temperature. Increasing temperature from 5 °C to 20 °C results in an increased amount of Voriconazole incorporated into the complex. The amount of Voriconazole per mole of HPβCD can be increased, if inclusion complex formation is performed at elevated temperatures.

Inclusion complexes formed at + 60 °C result in a molar ratio of HPβCD to voriconazole of 2 : 1. Decreasing temperature results in an increased molar ratio of HPβCD to voriconazole ending at ∼ 3:1 when inclusion complex formation is performed at 5 °C. Therefore, stochiometry of complex can be controlled by adjusting proper temperature conditions during complex formation.

### Example 3: Storage stability

Two formulations were manufactured applying different manufacturing parameters. Solution 8 was manufactured applying "standard-manufacturing conditions" with molar ratio of HPβCD to voriconazole of 3:1 and at 20 °C. Solution 9 was manufactured with increased concentrations of voriconazole and a lower HPβCD to voriconazole ratio at 60 °C.

Solution 8 was manufactured by dissolving of 37.96 g HPβCD and 3.039 g Voriconazole in 250 mL of ultrapure water at ambient temperature. After 3 hours, Voriconazole was completely dissolved. Solution was made up to 300 mL. A pH of 5.66 was obtained. Solution 8 had a concentration of voriconazole of 10 mg/mL, a concentration of HPβCD of 125 mg/mL and a molar ratio of HPβCD to voriconazole of 3.1.

After sterile filtration, solution was split into 3 parts and stored at 20 - 25 °C (ambient temperature), 40 °C and 60 °C. Solutions were analysed after 6 hours of storage by HPLC, pH and colour of solution.

Solution 9 was manufactured by dissolving 106.3 g HPβCD and 10.64 g Voriconazole in 250 mL of ultrapure water at 60 °C. After 2.5 hours, Voriconazole was completely dissolved. Solution was made up to 300 mL. A pH of 5.72 was obtained. Solution 9 had a concentration of voriconazole of 35 mg/mL, a concentration of HPβCD of 350 mg/mL and a molar ratio of HPβCD to voriconazole of 2.5.

After sterile filtration (0.22 µm), solution was split into 3 parts and stored at 20 - 25 °C (ambient temperature), 40 °C and 60 °C. Solutions were analysed after 6 hours of storage by HPLC, pH and colour of solution.

After manufacturing, solutions were splitted and individual solutions were stored at 20 °C, 40 °C and 60 °C.

The solutions were analysed regarding colour of solution, pH, assay and impurities.

For solution 8 and 9, no change of colour during storage at different temperatures was observed.

The pH was at the beginning at 5.8 for solution 8 or 6.0 for solution 9. During storage, only slight changes of pH could be observed. All pH-values were in the range of 5.8 - 6.1.

Assay values are summarized in table 5. All assay values are in the range of 91 % to 97 %. Surprisingly, assay values increase after 6 hours storage. No systematic decrease of impurities for storage at increased temperature could be observed.

**Table 5**

| Solution | Temperature | Time | Assay | Theoretical Assay | % |
|---|---|---|---|---|---|
| | [°C] | [hours] | Voriconazole | Voriconazole | Voriconazole |
| | | | [mg/mL] | [mg/mL] | |
| 8 | Start | 0 | 9.65 | 10.00 | 96.50 |
| 8 | 20 | 6 | 9.70 | 10.00 | 97.00 |
| 8 | 40 | 6 | 9.77 | 10.00 | 97.70 |
| 8 | 60 | 6 | 9.71 | 10.00 | 97.10 |
| 9 | Start | 0 | 32.09 | 35.00 | 91.70 |
| 9 | 20 | 6 | 33.38 | 35.00 | 95.40 |
| 9 | 40 | 6 | 32.94 | 35.00 | 94.10 |
| 9 | 60 | 6 | 33.11 | 35.00 | 94.60 |

## Claims

1. A process for improving the solubility of voriconazole in aqueous solutions comprising
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

2. The process of claim 1, wherein the temperature in step (ii) is in the range of from 40 °C to 60 °C.

3. The process of any of claims 1 or 2, wherein in step (ii) the solution comprising voriconazole and a beta-cyclodextrin is kept at a temperature of at least 40 °C for a time of from 1 minute to 24 hours.

4. The process of any of claims 1 to 3, wherein the process comprises an additional step (iii):
(iii) lyophilisation of the composition obtained in step (ii).

5. The process of any of claims 1 to 4, wherein the molar ratio of voriconazole and a beta-cyclodextrin in the composition is in the range of from 2.5 to 3.75.

6. The process of any of claims 1 to 5, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins.

7. The process of any of claims 1 to 6, wherein the beta-cyclodextrin is a hydroxyalkylated beta-cyclodextrin.

8. The process of any of claims 1 to 7, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

9. A process for preparing a composition comprising voriconazole and a beta-cyclodextrin, said process comprising the steps of
(i) preparing an aqueous solution of voriconazole and a beta-cyclodextrin;
(ii) keeping the solution obtained in step (i) comprising voriconazole and a beta-cyclodextrin at a temperature of at least 40 °C to obtain a composition comprising voriconazole and a beta-cyclodextrin.

10. The process according to claim 9, wherein the temperature in step (ii) is in the range of from 40 °C to 60 °C.

11. The process according to any of claims 9 or 10, wherein in step (ii) the solution comprising voriconazole and a beta-cyclodextrin is kept at a temperature of at least 40 °C for a time of from 1 minute to 24 hours.

12. The process according to any of claims 9 to 11, wherein the process comprises an additional step (iii):
(iii) lyophilisation of the composition obtained in step (ii).

13. The process according to any of claims 9 to 12, wherein the molar ratio of voriconazole and a beta-cyclodextrin in the composition is in the range of from 2.5 to 3.75.

14. The process according to any of claims 9 to 13, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins.

15. The process according to any of claims 9 to 14, wherein the beta-cyclodextrin is a hydroxyalkylated beta-cyclodextrin.

16. The process according to any of claims 9 to 15, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

17. A composition comprising voriconazole and a beta-cyclodextrin obtainable by a process according to any of claims 1 to 16.

18. The composition of claim 17, wherein the molar ratio of voriconazole and a beta-cyclodextrin in the composition is in the range of from 2.5 to 3.75.

19. The composition of any of claims 17 to 18, wherein the beta-cyclodextrin is selected from the group of beta-cyclodextrin, hydroxyalkylated beta-cyclodextrins, branched beta-cyclodextrins, methylated beta-cyclodextrins, ethylated beta-cyclodextrins and anionic beta-cyclodextrins.

20. The composition of any of claims 17 to 19, wherein the beta-cyclodextrin is a hydroxyalkylated beta-cyclodextrin.

21. The composition of any of claims 17 to 20, wherein the beta-cyclodextrin is a hydroxyalkylated beta-cyclodextrin selected from the group of 2-hydroxyethyl-beta-cyclodextrin, 2-hydroxypropyl-beta-cyclodextrin, 3-hydroxypropyl-beta-cyclodextrin and 2,3-dihydroxypropyl-beta-cyclodextrin.

22. A method of treating a fungal infection comprising administering to a patient in need of such treating a therapeutically effective amount of the composition comprising voriconazole and a beta-cyclodextrin according to any of claims 17 to 21 or a composition obtainable according to a process according to any of claims 1 to 16 and at least one pharmaceutically acceptable excipient.

23. A pharmaceutical composition comprising the composition comprising voriconazole and a beta-cyclodextrin according to any of claims 17 to 21 or a composition obtainable according to a process according to any of claims 1 to 16 and at least one pharmaceutically acceptable excipient.

24. A composition according to claim 23 for treating a fungal infection in a mammal, in particular in man.

25. Use of a therapeutically effective amount of the composition comprising voriconazole and a beta-cyclodextrin according to any of claims 17 to 21 or a composition obtainable according to a process according to any of claims 1 to 16 and at least one pharmaceutically acceptable excipient for the preparation of a medicament for treating a fungal infection, preferably wherein said medicament is to be administered to a patient in need thereof.
